Europäisches Patentamt

European Patent Office

Office européen des brevets

(11) Publication number: **0 051 167**
**B1**

(12) **EUROPEAN PATENT SPECIFICATION**

(45) Date of publication of patent specification: **05.12.84**

(21) Application number: **81108152.0**

(22) Date of filing: **09.10.81**

(51) Int. Cl.³: **A 61 B 10/00, A 61 B 1/00**

(54) **Endoscope fitted with an ultrasonic diagnostic device.**

(30) Priority: **28.10.80 JP 150978/80**

(43) Date of publication of application:
**12.05.82 Bulletin 82/19**

(45) Publication of the grant of the patent:
**05.12.84 Bulletin 84/49**

(84) Designated Contracting States:
**AT BE CH DE FR GB IT LI NL SE**

(56) References cited:
**EP-A-0 028 825**
**DE-A-2 950 203**
**DE-A-3 009 482**
**FR-A-2 467 583**

(73) Proprietor: **OLYMPUS OPTICAL CO., LTD.**
**43-2, 2-chome, Hatagaya Shibuya-ku**
**Tokyo 151 (JP)**

(72) Inventor: **Baba, Kazuo**
**1079 Kitano-machi**
**Hachioji-shi Tokyo (JP)**

�</4 Representative: **Prüfer, Lutz H., Dipl.-Phys.**
**Willroiderstrasse 8**
**D-8000 München 90 (DE)**

Courier Press, Leamington Spa, England.

## Description

This invention relates to an endoscope fitted with an ultrasonic diagnostic device.

This type of endoscope applied to date comprises an ultrasonic transceiving section formed at the distal end portion of the insertion section of the endoscope, and an observation optical system set in the proximity of said transceiving section as viewed from the axis of the insertion section, thereby enabling a visual observation to be made in the same direction as that in which ultrasonic diagnosis is carried out. Therefore, the conventional endoscope of the above-mentioned type has the drawback that where the ultrasonic transceiving section is tightly attached to the inner wall of, for example, the affected coeliac spot of a patient, then the observation window is also tightly set on said inner wall, failing to carry out a visual observation. To assure, therefore, a broad view field of the attachment of the ultrasonic transceiving section to the inner wall of the aforesaid affected coeliac spot, an endoscope has been proposed as described in DE—A—3009482 which comprises an observation window and ultrasonic transceiving section held in an expandable and contractible transparent balloon filled with a transparent liquid. However, this proposed endoscope has the drawbacks that since a view field is provided through the balloon, considerable difficulties arise in observation, if the ultrasonic transceiving section fails to be tightly attached to the affected coeliac spot; the externally effected expansion and contraction of the balloon consumes time; and the endoscope as a whole is complicated in arrangement.

It is accordingly the object of this invention to provide an endoscope provided with an ultrasonic diagnostic device, which assures a sufficiently broad view field even when the peripheral wall of the distal end portion of the insertion section is tightly attached to the inner wall of the affected coeliac cavity of a patient for ultrasonic diagnosis, and enables a spot requiring diagnose to be easily determined.

To attain the above-mentioned object, this invention provides an endoscope fitted with an ultrasonic diagnostic device, which comprises:

an insertion section to be introduced into a coeliac cavity of a human body;

an ultrasonic oscillator set in the proximity of the distal end of the insertion section; and an observation window,

the improvement being that the observation window is circumferentially displaced with respect to the mean transceiving direction of the ultrasonic transceiver such that the viewing direction and the mean transceiving direction make an angle with each other.

This invention can be more fully understood from the following detailed description when taken in conjunction with the accompanying drawings, in which:

Figs. 1 to 3 jointly show an endoscope according to one embodiment of this invention which is fitted with an ultrasonic diagnostic device: Fig. 1 is a lateral view of the whole of said endoscope; Fig. 2 is a longitudinal sectional view of the distal end of the insertion section; and Fig. 3 is a cross sectional view on line III—III of Fig. 2;

Fig. 4 schematically illustrates the manner in which the subject endoscope is applied; and

Fig. 5 schematically shows the manner in which an endoscope according to another embodiment of the invention is applied.

Description is now given with reference to the appended drawing of the endoscope according to one embodiment of this invention which is fitted with an ultrasonic diagnostic device.

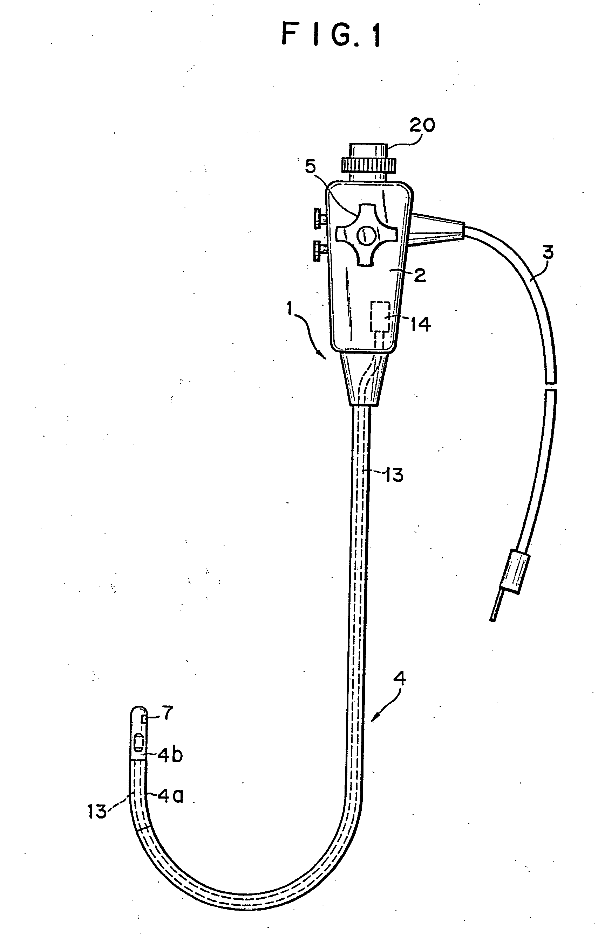

Referring to Fig. 1, reference numeral 1 denotes an endoscope, which comprises a control section 2, universal cord 3 for connecting the control section to an external power-light source device, and elongated flexible insertion section 4 which is taken into a coeliac cavity of a human body and whose proximal end is connected to the control section 2. That part of the insertion section 4 which lies close to its distal end is constituted by a bending portion 4a. This bending portion 4a can be freely flexed by an operation wire (not shown) when the bending knob 5 is actuated. The end of the bending portion 4a is fitted with a distal end portion 4b.

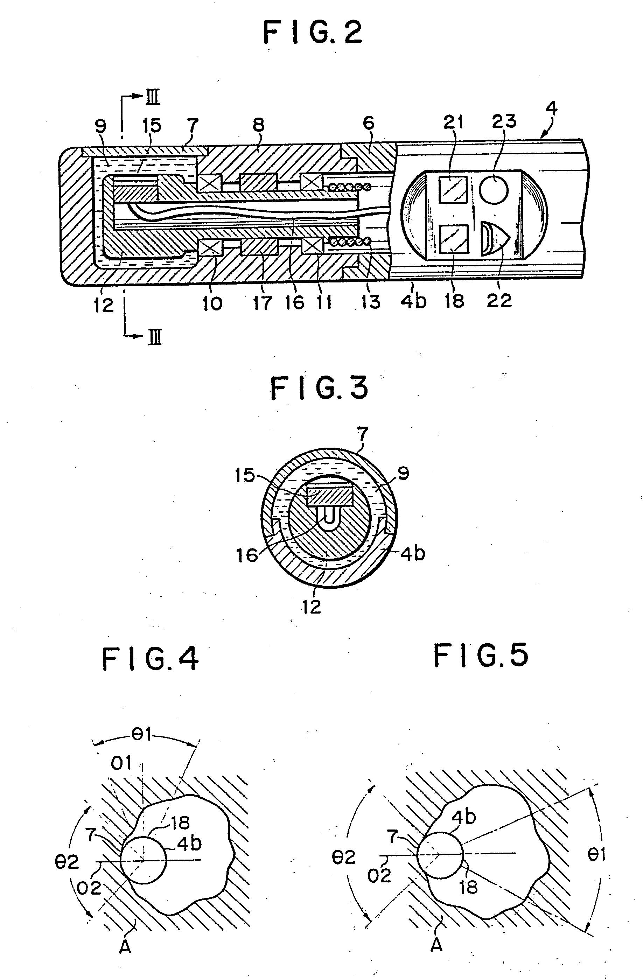

Description is now given with reference to Figs. 2 and 3 of the arrangement and operation of the distal end portion 4b. Referring to Fig. 2, reference numeral 6 denotes a metal member which is open at both ends and whose outer end portion is made cylindrical. The opening of a cylindrical cap 8 is pressed against the cylindrical outer end portion of the metal member 6. Thus, both members 6, 8 are concentrically fitted to each other. An ultrasonic transceiving window 7 is formed in part of the peripheral wall of the cap 8. A liquid-tight oscillator chamber 9 defined by the peripheral wall of the cap 8 is filled with a liquid acting as a medium for preparation of ultrasonic waves. A pair of bearings 10, 11 are fitted to the inner wall of the cap 8 in a state spaced from each other at a prescribed interval in the axial direction of the distal end portion of the insertion section 4. A cylindrical oscillator holder 12 is concentrically held in the cap 8 in a state rotatable about its axis by means of said paired bearings 10, 11. The open proximal end of the oscillator holder 12 is concentrically connected to the end of a flexible tube 13 like a tightly wound coil. The other end portion of the flexible tube 13 extends through the insertion section 4 to be coupled to the output shaft of a motor 14 shown in Fig. 1. A through hole is formed in the proximity of the outer end portion of the peripheral wall of the oscillator holder 12 in a state facing the ultrasonic transceiving window 7. An ultrasonic transceiver 15 (hereafter simply referred to as an oscillator 15) is securely held in said through hole. The ultrasonic oscillator 15 is designed to transmit and receive ultrasonic waves crosswise of the cap 8. The ultrasonic oscillator 15 is connected to one end of a signal cable 16, the other end portion of the signal cable 16 extends through the oscillator holder 12 and flexible tube 13 to the insertion section 4 and then to the control section 2, and is connected to the power-light source device by means of the universal cord 3. An ultrasonic drive signal is supplied to the oscillator 15 through the signal cable 16. An echo wave received by the oscillator 15 is transmitted to the control section 2.

A rotation angle detector 17 is set between the inner peripheral wall of the cap 8 and the outer peripheral wall of the oscillator holder 12. Said rotation angle detector 17 detects the rotated position of the oscillator holder 12. The direction in which ultrasonic scanning is to be made can be recognized from data on the rotation angle of the oscillator holder 12 which is furnished by said rotation angle detector 17. An observation window 18 is formed in part of the peripheral wall of the metal member 6 of the distal end portion 4b. With the foregoing embodiment, the observation window 18 has its center displaced from that of the ultrasonic transceiving window 7 at a prescribed angle of 90° clockwise or counterclockwise as measured from the circumferential direction of the metal part 6. The observation window 18 is optically connected to the control section 2 by the known optical medium means. The optical medium means is held, for example, in the distal end portion 4b in a state facing the observation window 18. Said optical medium means comprises a group of object lenses for receiving light beams supplied through the observation window 18 and an optical fiber bundle for conducting light means delivered from the group of the object lenses to the eyepiece 20 of the control section 2. An illumination window 21 is formed in part of the peripheral wall of the distal end metal member 6 at a point adjacent to the observation window 18. An illumination light beam is sent forth from the power-light source device to the illumination window 21 through an illumination optical bundle (not shown) extending through the insertion section 4. An air-water nozzle 22 used in washing the observation window 18 and a suction port 23 for drawing off waste liquids from the coeliac cavity are provided near the observation window 18 formed in the distal end metal member 6.

Description is now given with reference to Fig. 4 of the ultrasonic diagnosis by an endoscope arranged as described above. The insertion section 4 is taken into the coeliac cavity of a human body. While said coeliac cavity is observed by the eye through the observation window 18, with viewing range $\theta_1$, the distal end portion 4b is set near the spot of the inner wall A which is to be diagnosed. The central part of the ultrasonic transceiving window 7 is tightly abutted on said inner wall A by controlling the extent to which the bending portion 4a is flexed. At this time, the observation window 18 is not tightly abutted on the inner wall A of the coeliac cavity, but is deflected therefrom at a certain angle, thereby assuring the desired view of said observation window 18, and consequently allowing for the easy naked eye observation of the coeliac cavity. Later, the motor 14 is driven to rotate the flexible tube 13, thereby causing the oscillator holder 12 and ultrasonic oscillator 15 to be jointly rotated. Ultrasonic waves are issued from the oscillator 15 by actuating the ultrasonic transceiving circuit of the power-light source device. Echoes from the inner wall A of the coeliac cavity are received within angle $\theta_2$. An ultrasonic tomographic image based on the signal received and data on the rotated position of the observation window 18 detected by the rotation angle detector 17 is indicated on a monitor device.

With the foregoing embodiment, the observation window 18 is deflected at an angle of 90° from the ultrasonic transceiving window 7 as viewed from the circumferential direction of said ultrasonic transceiving window 7. Even during the ultrasonic diagnosis, therefore, the observation window 18 is prevented from being tightly abutted on the inner wall A of the coeliac cavity, thereby assuring a wide view field, and consequently enabling an object spot of ultrasonic diagnosis to be easily recognized and allowing for the accurate ultrasonic diagnosis by an unobstructed naked eye observation.

With the foregoing embodiment, the observation window 18 is deflected from the direction in which ultrasonic diagnosis is to be effected at an angle of 90° as measured from the circumferential direction of the ultrasonic transceiving window 7. However, said deflection angle need not be limited to the above level, but it is possible to deflect the observation window 18 from the direction of the ultrasonic diagnosis at an angle of, for example, 180° as shown in Fig. 5. However, a deflection angle ranging from 5° to 180° is preferred for the satisfactory visual observation of the inner wall A of the coeliac cavity. Further, the ultrasonic oscillator need not be rotated, but may be immovably set in the distal end portion of the insertion section.

## Claims

1. An endoscope provided with an ultrasonic diagnostic device, which comprises:
an insertion section (4) for insertion into the coeliac cavity (A) of a human body; and
an ultrasonic transceiver (15) and an observation window (18) adjacent the distal end (4b) of the insertion section (4), characterized in that the observation window (18) is circumferentially displaced with respect to the mean transceiving direction of the ultrasonic transceiver (15) such that the viewing direction and the mean transceiving direction make an angle with each other.

2. The endoscope according to claim 1, characterized in that the angle ranges from 5° to 180°.

3. The endoscope according to claim 2, characterized in that the angle is 90°.

## Patentansprüche

1. Endoskop mit eingebautem Ultraschall-Diagnostik-Gerät mit einem Einführungsteil (4) zur Einführung in die Bauchhöhle (A) eines menschlichen Körpers, und einem Ultraschall-Sende-Emfpangs-Gerät (15) und einem Beobachtungsfenster (18) benachbart dem vorderen Ende (4b) des Einführungsteils (4), dadurch gekennzeichnet, daß das Beobachtungsfenster (18) am Umfang bezüglich der mittleren Sende-Empfangs-Richtung des Ultraschall-Sende-Empfangs-Geräts (15) so versetzt ist, daß die Betrachtungseinrichtung und die mittlere Sende-Empfangs-Richtung miteinander einen Winkel bilden.

2. Endoskop nach Anspruch 1, dadurch gekennzeichnet, daß der Winkel im Bereich von 5° bis 180° ist.

3. Endoskop nach Anspruch 2, dadurch gekennzeichnet, daß der Winkel 90° ist.

## Revendications

1. Endoscope comportant un appareil de diagnostic par ultrasons, comprenant:

uné partie introduction (4) à introduire dans la cavité coeliaque (A) d'un corps humain; et

un émetteur-récepteur d'ultrasons (15) et une fenêtre d'observation (18) au voisinage de l'extrémité distale (4b) de la partie introduction (4), caractérisé en ce que la fenêtre d'observation (18) est décalée circonférentiellement par rapport à la direction moyenne d'émission-réception de l'émetteur-récepteur d'ultrasons (15) de telle manière que la direction de l'observation visuelle et la direction moyenne d'émission-réception fassent un angle entre elles.

2. Endoscope selon la revendication 1, caractérisé en ce que l'angle est compris entre 5° et 180°.

3. Endoscope selon la revendication 2, caractérisé en ce que l'angle est de 90°.

# F I G. 1

# FIG. 2

# FIG. 3

# FIG. 4

# FIG. 5